# EUROPÄISCHE PATENTANMELDUNG

(11) **EP 3 147 663 A2**
(43) Veröffentlichungstag der Anmeldung: **29.03.2017**
(21) Anmeldenummer: 16192464.2
(22) Anmeldetag: 03.09.2008
(51) Int. Cl.: G01N 33/564

(54) **MARKERSEQUENZEN FÜR MULTIPLE SKLEROSE UND DEREN VERWENDUNG**

(30) Priorität: 03.09.2007 DE 102007041657
(62) Teilanmeldung aus: 16164136.0
(71) Anmelder: Protagen AG, 44227 Dortmund (DE)
(72) Erfinder: BEATOR, Jens, 13507 Berlin (DE); KOWALD, Axel, 44892 Bochum (DE); LÜKING, Angelika, 44892 Bochum (DE); MEYER, Helmut E., 45661 Recklinghausen (DE)
(74) Vertreter: Simandi, Claus

(57) **Zusammenfassung**

Die vorliegende Erfindung betrifft neue Markersequenzen für Multiple Sklerose und deren diagnostische Verwendung samt einem Verfahren zum Screenen von potentiellen Wirkstoffen für Multiple Sklerose mittels dieser Markersequenzen. Ferner betrifft die Erfindung eine diagnostische Vorrichtung enthaltend solche Markersequenzen für Multiple Sklerose, insbesondere ein Proteinbiochip und dessen Verwendung.

## Beschreibung

Die vorliegende Erfindung betrifft neue Markersequenzen für Multiple Sklerose und deren diagnostische Verwendung samt einem Verfahren zum Screenen von potentiellen Wirkstoffen für Multiple Sklerose mittels dieser Markersequenzen. Ferner betrifft die Erfindung eine diagnostische Vorrichtung enthaltend solche Markersequenzen für Multiple Sklerose, insbesondere ein Proteinbiochip und dessen Verwendung.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliothek konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Es besteht jedoch ein hohes Bedürfnis indikationsspezifische diagnostische Vorrichtungen, wie einen Proteinbiochip, bereitzustellen.

Markersequenzen und deren diagnostischen Verwendung für die Multiple Sklerose, insbesondere in der Ausführungsform eines Proteinbiochips sowie diesbezügliche Tests für das Screening von Wirkstoffen sind im Stand der Technik nicht beschrieben.

Die Aufgabe der vorliegenden Erfindung ist daher die Bereitstellung von Markersequenzen und deren diagnostischen Verwendung.

Die Bereitstellung von spezifischen Markersequenzen erlaubt eine sichere Diagnose und Stratifizierung von Patienten mit Multiple Sklerose, insbesondere mittels eines Proteinbiochips.

Daher betrifft die Erfindung die Verwendung von Markersequenzen zur Diagnose von Multiple Sklerose, wobei mindestens eine Markersequenz einer cDNA ausgewählt aus der Gruppe SEQ 1 - 395 oder jeweils ein dafür kodierendes Protein oder jeweils einer Teilsequenz oder Fragment davon (nachstehend: erfindungsgemäße Markersequenzen) an oder von einem zu untersuchenden Patienten bestimmt wird.

Die erfindungsgemäßen Markersequenzen konnten mittels differentiellem Screenen von Proben von gesunden Probanden mit Patientenproben mit Multiple Sklerose identifiziert werden.

Der Begriff "Multiple Sklerose ((MS), auch Encephalomyelitis disseminata)" ist z.B. nach Pschyrembel, de Gruyter, 261. Auflage (2007), Berlin definiert und betrifft eine autoimmunentzündliche/demyelinisierende und degenerative Erkrankung des Erkrankung des Zentralnervensystems.

In einer weiteren Ausführungsform werden mindestens 2 bis 5 oder 10, vorzugsweise 30 bis 50 Markersequenzen oder 50 bis 100 oder mehr Markersequenzen an oder von einem zu untersuchenden Patienten bestimmt.

In einer besonderen Ausführungsform der Erfindung sind die Markersequenzen der SEQ 1-20 besonders bevorzugt, bevorzugt sind die Markersequenzen SEQ 21-50 und weiterhin bevorzugt sind die Markersequenzen SEQ 51 - 100.

In einer weiteren Ausführungsform der Erfindung sind jeweils besonders bevorzugt die Markersequenzen SEQ 1-10 und SEQ 11-20, sowie bevorzugt SEQ 21-30, SEQ 31-40 oder SEQ 41-50.

In einer weiteren Ausführungsform der Erfindung können die erfindungsgemäßen Markersequenzen ebenfalls mit bekannten Biomarker für diese Indikation kombiniert, ergänzt, fusioniert oder erweitert werden.

In einer bevorzugten Ausführungsform erfolgt die Bestimmung der Markersequenzen außerhalb des menschlichen Körpers und die Bestimmung erfolgt in einer ex vivo / in vitro Diagnose.

In einer weiteren Ausführungsform der Erfindung betrifft die Erfindung die Verwendung von Markersequenzen als Diagnostika, wobei mindestens eine Markersequenz einer cDNA ausgewählt aus der Gruppe SEQ 1 - 395 oder jeweils ein dafür kodierendes Protein oder jeweils einer Teilsequenz oder Fragment davon ist.

Ferner betrifft die Erfindung ein Verfahren zur Diagnose von Multiple Sklerose, wobei a.) mindestens eine Markersequenz einer cDNA ausgewählt aus der Gruppe SEQ 1 - 395 oder jeweils ein dafür kodierendes Protein oder jeweils einer Teilsequenz oder Fragment davon auf einem festen Träger aufgebracht wird und b.) mit Körperflüssigkeit oder Gewebeauszug eines Patienten in Kontakt gebracht wird und c.) der Nachweis einer Wechselwirkung der Körperflüssigkeit oder Gewebeauszug mit den Markersequenzen aus a.) erfolgt.

Daher betrifft die Erfindung ebenfalls Diagnostika zur Diagnose von Multiple Sklerose jeweils ausgewählt aus der Gruppe SEQ 1 - 395 oder jeweils ein dafür kodierendes Protein oder jeweils einer Teilsequenz oder Fragment davon.

Der Nachweis einer solchen Wechselwirkung kann beispielsweise durch eine Sonde, insbesondere durch einen Antikörper erfolgen.

Daher betrifft die Erfindung ebenfalls die Aufgabe eine diagnostische Vorrichtung oder einen Assay, insbesondere einen Proteinbiochip, bereitzustellen, der für die Multiple Sklerose eine Diagnose oder Untersuchung erlaubt.

Ferner betrifft die Erfindung ein Verfahren zum Stratifizieren, insbesondere zur Risikostratifizierung und / oder Therapiesteuerung eines Patienten mit Multiple Sklerose, wobei mindestens eine Markersequenz einer cDNA ausgewählt aus der Gruppe SEQ 1 - 395 oder jeweils ein dafür kodierendes Protein an einem zu untersuchenden Patienten bestimmt wird.

Ferner umfasst ist die Stratifizierung der Patienten mit Multiple Sklerose in neue oder etablierte Subgruppen der Multiple Sklerose, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

"Diagnose" im Sinne dieser Erfindung bedeutet die positive Feststellung der Multiple Sklerose mittels der erfindungsgemäßen Markersequenzen sowie die Zuordnung der Patienten zu der Multiple Sklerose. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderen Krankheiten vonnöten sein. Daher umfasst der Begriff Diagnose ebenfalls die Differentialdiagnose der Multiple Sklerose mittels der erfindungsgemäßen Markersequenzen sowie die Prognose der Multiple Sklerose. "Stratifizieren (auch: Stratifikation) oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Multiple Sklerose untersucht wird.

Der Begriff "Markersequenzen" im Sinne dieser Erfindung bedeutet, dass die cDNA oder das jeweils daraus erhältliche Polypeptid oder Protein signifikant für Multiple Sklerose sind. Beispielsweise können die cDNA oder das jeweils daraus erhältliche Polypeptid oder Protein eine Wechselwirkung mit Substanzen aus der Körperflüssigkeit oder Gewebeauszug eines Patienten mit Multiple Sklerose aufweisen (z.B. Antigen (Epitop) / Antikörper (Paratop) Wechselwirkung). Im Sinne der Erfindung bedeutet "wobei mindestens eine Markersequenz einer cDNA ausgewählt aus der Gruppe SEQ 1 - 395 oder jeweils ein dafür kodierendes Protein oder jeweils einer Teilsequenz oder Fragment davon an einem zu untersuchenden Patienten bestimmt wird", dass eine Wechselwirkung zwischen der Körperflüssigkeit oder Gewebeauszuges eines Patienten und den erfindungsgemäßen Markersequenzen nachgewiesen wird. Eine solche Wechselwirkung ist z.B. eine Bindung, insbesondere eine bindende Substanz an mindestens einer erfindungsgemäßen Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

In einer weiteren Ausführungsform der Erfindung können jedoch die erfindungsgemäßen Markersequenzen in einer signifikant höheren oder niedrigeren Expressionsrate oder Konzentration vorliegen, dass auf die Multiple Sklerose hinweist. Hierbei wird mittels Proteomics oder Nukleinsäureblots die relativen Expressionsraten krank / gesund der erfindungsgemäßen Markersequenzen für Multiple Sklerose bestimmt.

Die Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen sind Gegenstand der Tabelle A und können durch den jeweilig zitierten Datenbankeintrag (auch mittels Internet:
http://www.ncbi.nlm.nih.gov/) eindeutig identifiziert werden (siehe in Tabelle A: dort Accession No.).

Erfindungsgemäß umfassen die Markersequenzen auch solche Modifikationen der cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

In einer weiteren Ausführungsform der Erfindung sind ebenfalls Teilsequenzen oder Fragmente der erfindungsgemäßen Markersequenzen umfasst. Insbesondere solche Teilsequenzen, die eine Identität von 95%, 90 %, insbesondere 80% oder 70 % mit den erfindungsgemäßen Markersequenzen aufweisen.

In einer weiteren Ausführungsform kann die jeweilige Markersequenz in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Ein weiterer Gegenstand der Erfindung betrifft eine Anordnung von Markersequenzen enthaltend mindestens eine Markersequenz einer cDNA ausgewählt aus der Gruppe SEQ 1 - 395 oder jeweils ein dafür kodierendes Protein. Vorzugsweise enthält die Anordnung mindestens 2 bis 5 oder 10, vorzugsweise 30 bis 50 Markersequenzen oder 50 bis 100 oder mehr Markersequenzen.

Im Rahmen dieser Erfindung bedeutet "Anordnung" synonym "Array" und sofern dieser "Array" zur Identifizierung von zu Substanzen an Markersequenzen verwendet wird, ist hierunter ein "Assay" oder diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Proteinbindern erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA (z.B.einzelne Wells einer Mikrotiterplatte werden mit den erfindungsgemäßen Markersequenzenoder Kombinationen von Markersequenzen beschichtet, ggfs. robotergestützt in die einzelnen Wells der Mikrotiterplatte aufgebracht; Beispiele sind diagnostische ELISA-Kits der Fa. Phadia oder Multiplex-ELISA-Kits "Searchlight" der Fa. Pierce/Thermo Fisher Scientific), Beadbased Assay (spektral unterscheidbare bead-Populationen werden mit Markersequenzen/Kombinationen von Markersequenzen beschichtet. Die Patientenprobe wird mit dieser bead-Population inkubiert und gebundene (Auto-)-Antikörper werden mittels eines weiteren Floureszenzmarkierten Sekundärantikörper/Detektionsreagenz über Messung der Fluoreszenz nachgewiesen; z. B. Borrelia IgG-Kit oder Athena-Multilyte der Fa. Multimetrix), Line Assay (erfindungsgemäßen Markersequenzen oderKombinationen von Markersequenzen werden robotergestützt auf Membranen immobilisiert, welche mit der Patientenprobe untersucht/inkubiert werden; Beispiel "Euroline" der Fa. Euroimmun AG), Western Blot (Beispiel "Euroline-WB" der Fa. Euroimmun AG), immunchromatographische Verfahren (z.B. Lateral Flow Immunoassays; Markersequenzen/Kombinatione von Markersequenzen werden auf Teststreifen (Membranen, US 5,714,389 u.v.a) immobilisiert; Beispiel "One Step HBsAg" Test Device von Acon Laboratories) oder ähnliche immunologische Single- oder Multiplex-Nachweisverfahren.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip bestehend aus einer Anordnung enthaltend erfindungsgemäße Markersequenzen.

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden.

Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einen festen Träger fixiert, gespottet oder immobilisiert.

Daher betrifft die Erfindung eine Anordnung, wobei die Markersequenzen als Clone vorliegen.

Zusätzlich können die Markersequenzen in der jeweiligen Form in Form eines Fusionsproteins vorliegen, welches beispielsweise mindestens ein Affinitätsepitop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

Eine Markersequenz kann sich auch aus mehreren einzelnen Markersequenzen zusammensetzen. Dies kann die Klonierung einzelner Fragmente zu einem großen gemeinsamen Fragment und die Expression dieses kombinierten Fragmentes beinhalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz für Multiple Sklerose, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Ferner betrifft die Erfindung ein Verfahren zum Identifizieren und Charakterisieren einer Substanz für Multiple Sklerose, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Die zu untersuchende Substanz kann ein beliebiges natives oder nicht-natives Biomolekül, ein synthetisches chemisches Molekül, eine Mischung oder eine Substanzbibliothek sein.

Nachdem die zu untersuchende Substanz eine Markersequenz kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Multiple Sklerose entwickelt und erhältlich durch den Einsatz des erfindungsgemäßen Assays oder Proteinbiochip.

Daher betrifft die Erfindung ebenfalls die Verwendung einer erfindungsgemäßen Anordnung oder einem Assay zum Screenen von Wirkstoffen für Multiple Sklerose.

Daher betrifft die Erfindung in einer weiteren Ausführungsform ebenfalls ein Target zur Behandlung und Therapie von Multiple Sklerose jeweils ausgewählt aus der Gruppe SEQ 1 - 395 oder jeweils ein dafür kodierendes Protein.

In einer weiteren Ausführungsform betrifft die Erfindung ebenfalls die Verwendung der erfindungsgemäßen Markersequenzen, vorzugsweise in Form einer Anordnung, als Affinitätsmaterial zur Durchführung einer Apherese bzw. iwS. einer Blutwäsche, wobei Substanzen aus Körperflüssigkeiten eines Patienten mit Multiple Sklerose, wie Blut oder Plasma, an die erfindungsgemäßen Markersequenzen binden und folglich der Körperflüssigkeit selektiv entzogen werden können.

Beispiele und Figuren:
Zehn oder mehr Patientenproben wurden individuell gegen eine cDNA Expressionsbibliothek gescreent. Die Multiple Sklerosespezifischen Expressionsklone wurden ermittelt durch einen Vergleich mit zehn oder mehr gesunden Proben. Die Identität der Markersequenzen wurde durch DNA-Sequenzierung ermittelt.

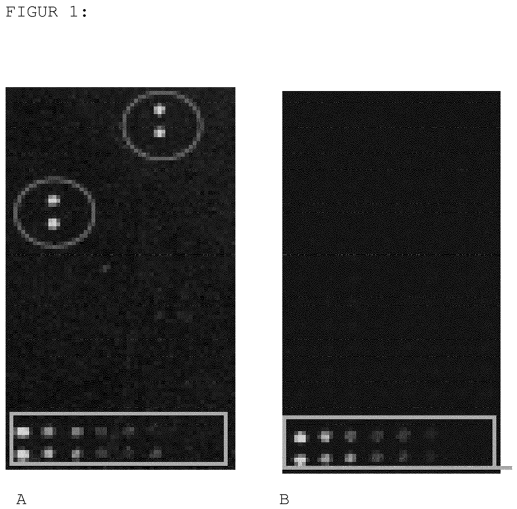

In Figur 1 wird das differentielle Screenen zwischen zwei Proteinbiochips aus jeweils einer cDNA-Expressionsbank eines Patienten und einem gesunden Probanden gezeigt. Die differentiellen Clone werden mittels Fluoresenzmarkierung nachgewiesen und bioinformatorisch ausgewertet.

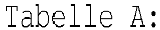

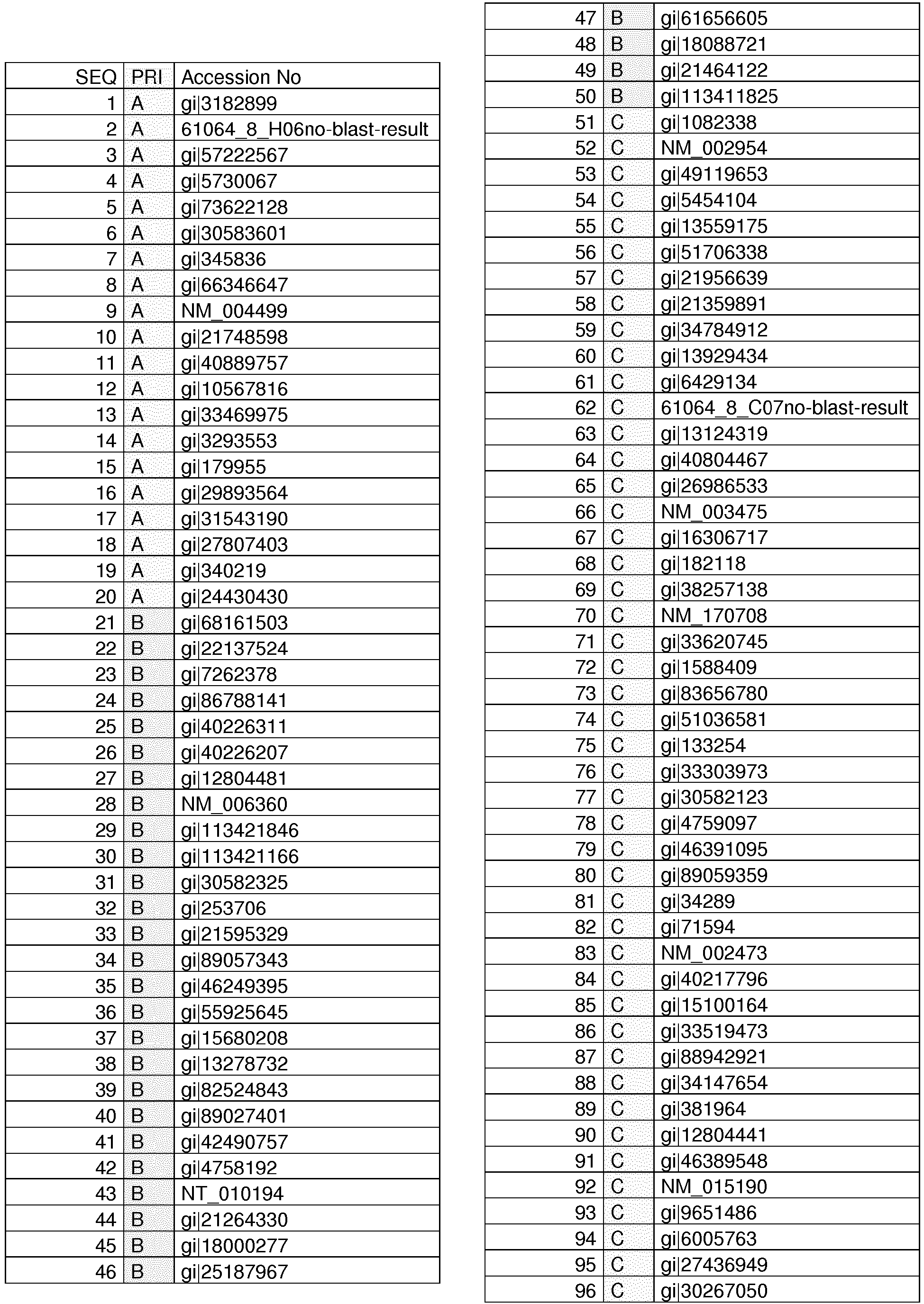

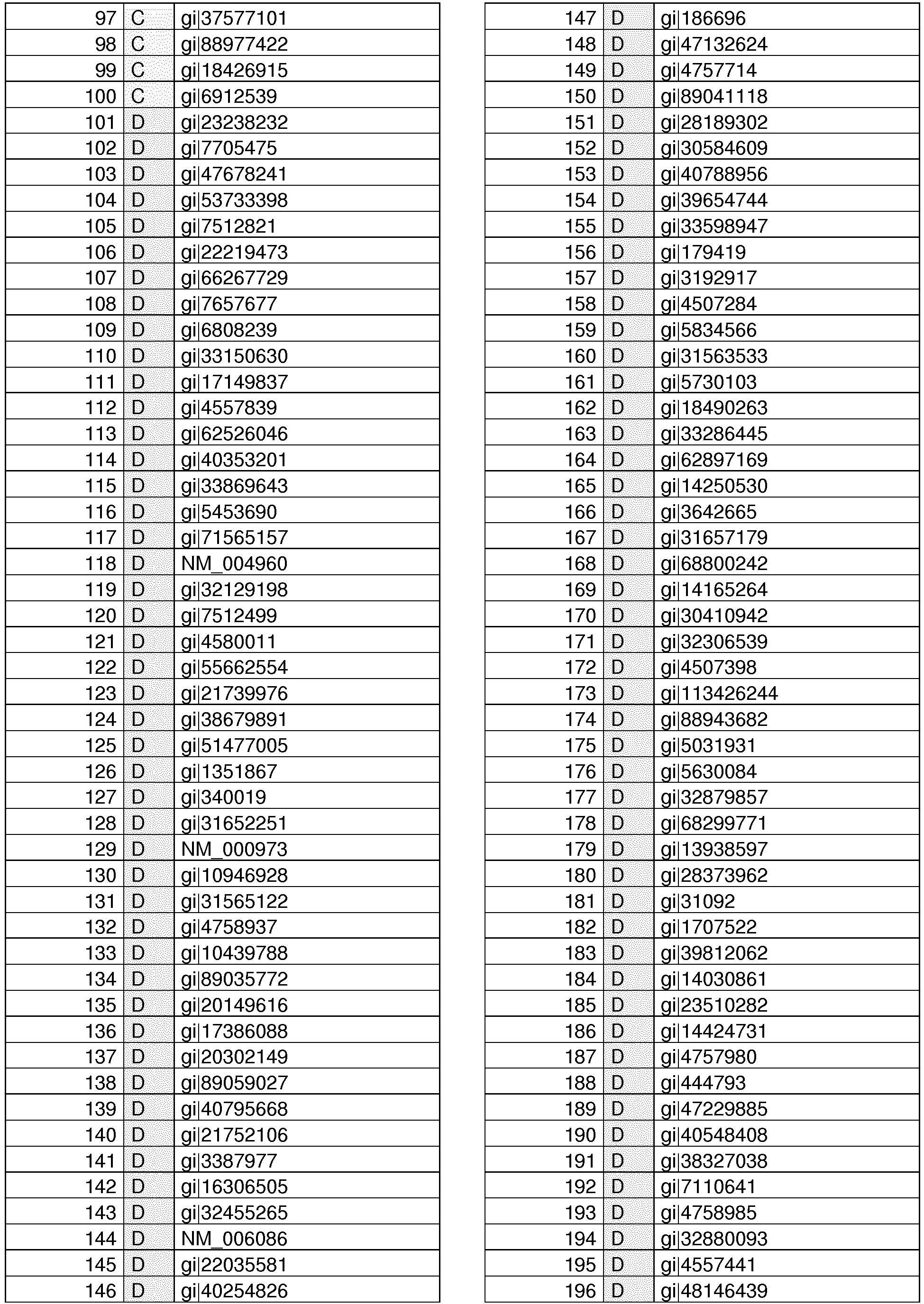

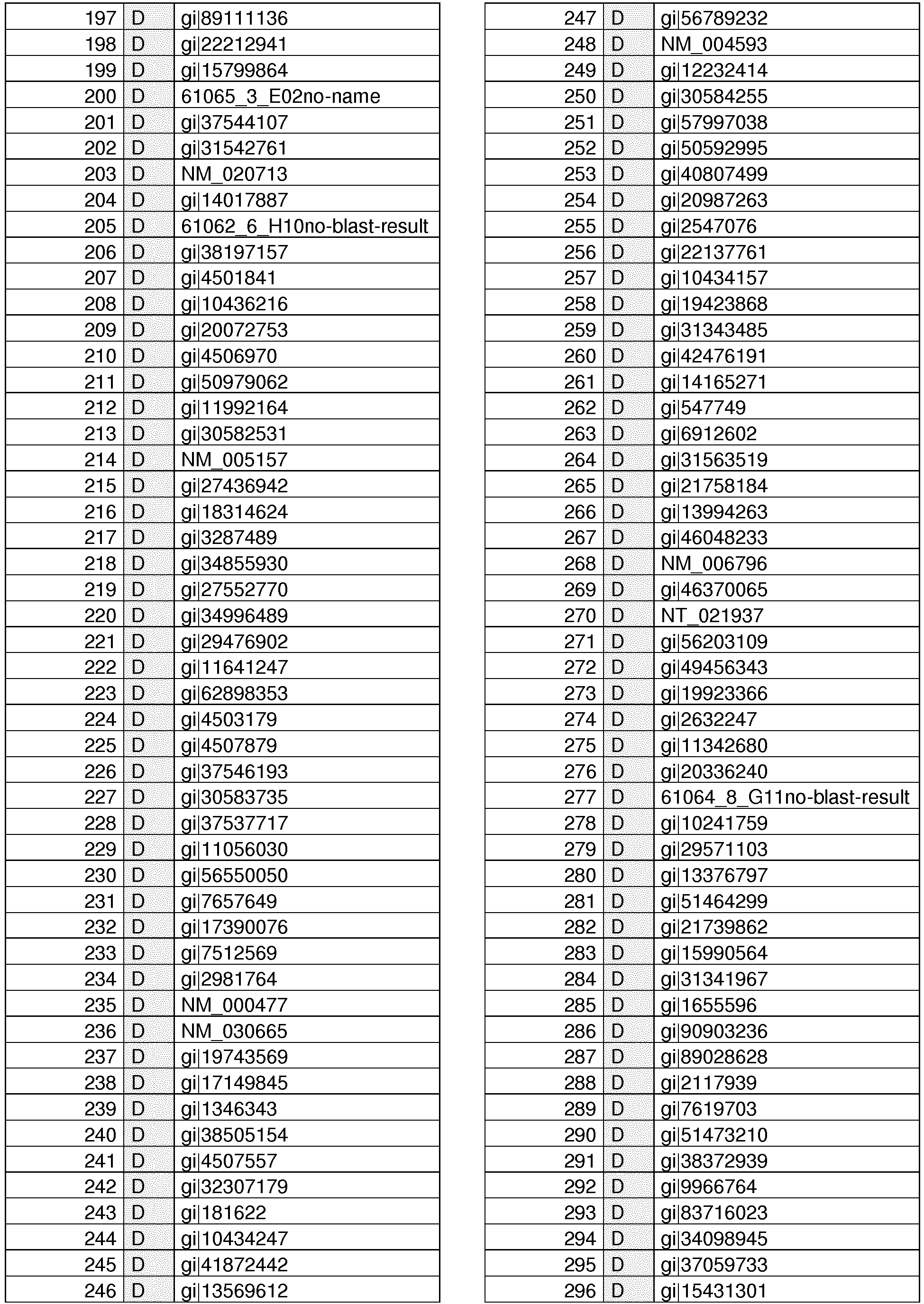

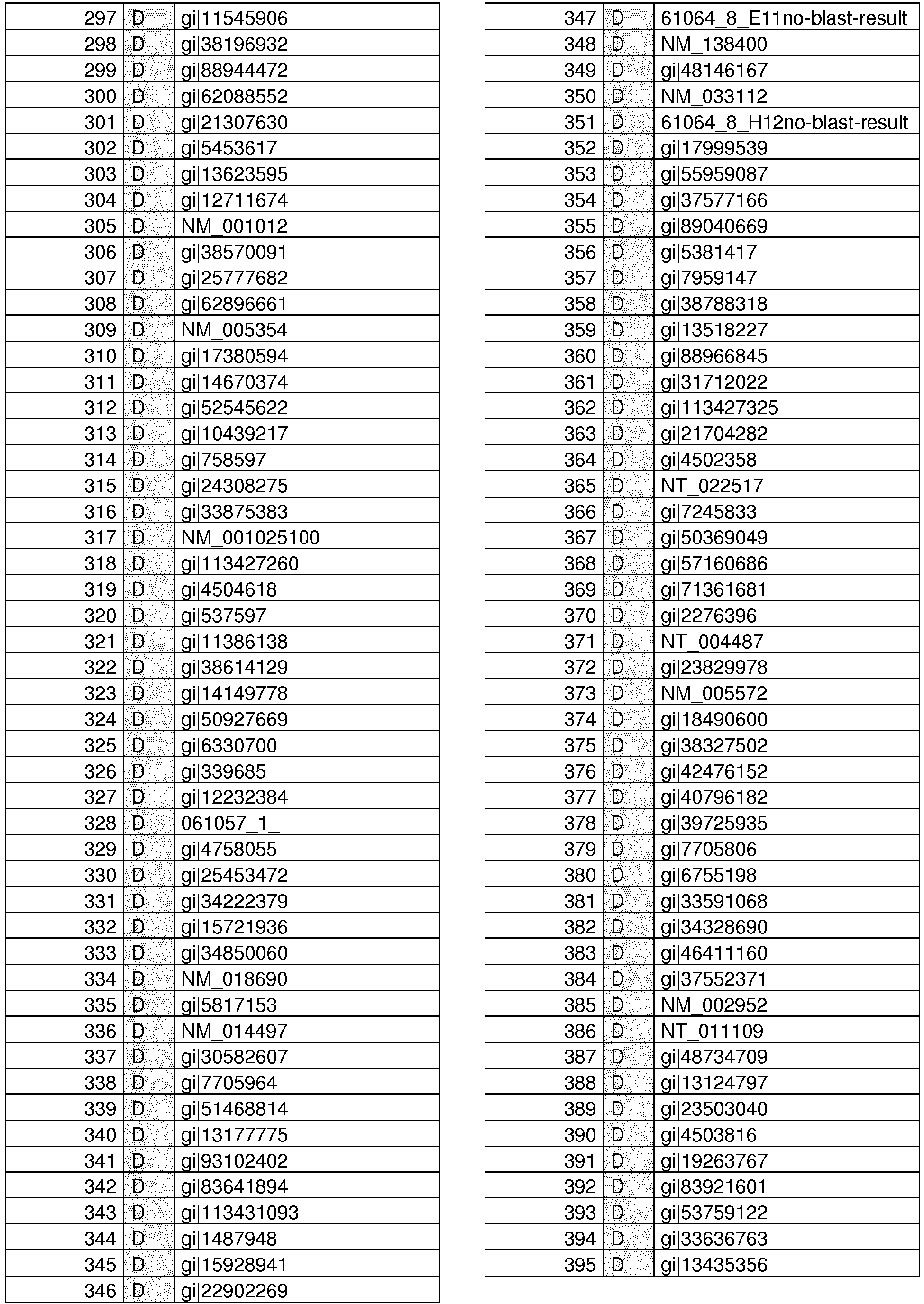

## Patentansprüche

1. Verwendung der Markersequenzen zur Diagnose von Multiple Sklerose, wobei mindestens eine Markersequenz einer cDNA ausgewählt aus der Gruppe SEQ ID. No. 279, SEQ ID No. 1 - 395 oder jeweils ein dafür kodierendes Protein oder jeweils einer Teilsequenz oder Fragment davon an oder von einem zu untersuchenden Patienten bestimmt wird.

2. Verwendung der Markersequenzen zur Diagnose von Multiple Sklerose nach Anspruch 1, **dadurch gekennzeichnet**, mindestens 2 bis 5 oder 10, vorzugsweise 30 bis 50 Markersequenzen oder 50 bis 100 oder mehr Markersequenzen an oder von einem zu untersuchenden Patienten bestimmt wird.

3. Verwendung der Markersequenzen zur Diagnose von Multiple Sklerose nach Anspruch 1, **dadurch gekennzeichnet, dass** die SEQ ID. No. 1-20 oder SEQ ID. No. 21-50 oder SEQ ID. No. 51- 100 oder jeweils ein dafür kodierendes Protein oder jeweils einer Teilsequenz oder Fragment davon an oder von einem zu untersuchenden Patienten bestimmt wird.

4. Verwendung der Markersequenzen zur Diagnose von Multiple Sklerose nach einem der vorhergehenden Ansprüche, **dadurch gekennzeichnet, dass** die Bestimmung mittels in-vitro Diagnose erfolgt.

5. Verwendung einer Markersequenz einer cDNA jeweils ausgewählt aus der Gruppe SEQ ID. No. 279, SEQ ID No. 1 - 395 oder jeweils ein dafür kodierendes Protein oder jeweils einer Teilsequenz oder Fragment davon als Diagnostikum.

6. Verwendung der Markersequenzen zur Diagnose von Multiple Sklerose nach einem der vorhergehenden Ansprüche, **dadurch gekennzeichnet, dass** die Markersequenzen auf einem festen Träger aufgebracht werden, insbesondere einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix.

7. Verfahren zur Diagnose von Multiple Sklerose, wobei
a.) mindestens eine Markersequenz einer cDNA ausgewählt aus der Gruppe SEQ ID. No. 279, SEQ ID No. 1 - 395 oder jeweils ein dafür kodierendes Protein oder jeweils einer Teilsequenz oder Fragment davon auf einem festen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

8. Verfahren zum Stratifizieren, insbesondere zur Risikostratifizierung, oder zur Therapiesteuerung eines Patienten mit Multiple Sklerose, wobei mindestens eine Markersequenz einer cDNA ausgewählt aus der Gruppe SEQ ID. No. 279, SEQ ID No. 1 - 395 oder jeweils ein dafür kodierendes Protein oder jeweils einer Teilsequenz oder Fragment davon an oder von einem zu untersuchenden Patienten bestimmt wird.

9. Verfahren nach Anspruch 8, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

10. Anordnung von Markersequenzen enthaltend mindestens eine Markersequenz einer cDNA ausgewählt aus der Gruppe SEQ ID. No. 279, SEQ ID No. 1 - 395 oder jeweils ein dafür kodierendes Protein.

11. Assay, Proteinbiochip bestehend aus einer Anordnung nach Anspruch 10, **dadurch gekennzeichnet, dass** die Markersequenzen auf einem festen Träger aufgebracht sind.

12. Verwendung einer Anordnung nach Anspruch 10 oder einem Assay nach Anspruch 11 zum Identifizieren und Charakterisieren einer Substanz für Multiple Sklerose enthaltend Mittel zum Nachweis eines Bindungserfolges, **dadurch gekennzeichnet, dass** eine Anordnung oder Assay mit a.) mindestens einer zu untersuchenden Substanz in Kontakt gebracht wird und b.) ein Bindungserfolg nachgewiesen wird.

13. Diagnostika zur Diagnose von Multiple Sklerose jeweils ausgewählt aus der Gruppe SEQ ID. No. 279, SEQ ID No. 1 - 395 oder jeweils ein dafür kodierendes Protein oder jeweils einer Teilsequenz oder Fragment davon.
